# EUROPEAN PATENT SPECIFICATION

(11) **EP 1 164 981 B1**
(45) Date of publication and mention of the grant of the patent: **27.10.2004**
(21) Application number: 00902721.0
(22) Date of filing: 04.02.2000
(51) Int. Cl.: A61F 5/37

(54) **IMPROVED RESTRAINING DEVICE**
VERBESSERTE FESSELUNGSVORRICHTUNG
DISPOSITIF DE CONTENTION AMELIORE

(30) Priority: 05.02.1999 GB 9902671
(43) Date of publication of application: 02.01.2002
(73) Proprietor: Fuller, Roger Stephen, Knaphill, Woking, Surrey GU21 2AW (GB)
(72) Inventor: Fuller, Roger Stephen, Knaphill, Woking, Surrey GU21 2AW (GB)
(86) International application number: PCT/GB2000/000341
(87) International publication number: WO 2000/045757

(56) References cited:
- GB-A- 1 188 628
- GB-A- 2 108 572
- US-A- 2 425 489
- US-A- 5 088 158

## Description

The present invention relates to an improved restraining device for use on a person in a medical or rescue situation, who is, for example, violent, potentially violent or panicking.

One of the main areas of use of restraining devices is by the police force, at present, the most common form of restraint is firstly physical and then many different kinds of handcuffs. The physical containment of a violent person is very difficult, and often restraining a violent person requires several officers, even when the person is placed on the ground. Restraining a violent person can be especially difficult if the person is wearing little or no garments, officers having very little to grab hold of to get them onto the ground.

Conventional restraining devices have involved simple straps usually with some form of moveable lockable clip, through which the strap can be pulled and secured. However, such conventional restraining devices are difficult to apply to the detainee, requiring physical contact with the strap to pull it over the detainee's head and around the upper torso and arms before it can be secured.

A restraining device comprising the features of the preamble of claim 1 is disclosed in document GB 2 108 572.

It is an object of the present invention to provide a restraining device which is easier to apply and position.

It is a second object of the present invention to provide a restraining device which is quick and easy to secure, and has a quick release mechanism.

It is a further object of the present invention to provide a restraining device which is applicable to many situations and may be easily modified for a particular situation.

It is a still further object of the present invention to provide a restraining device that is easy to store or carry on the body.

According to the present invention there is provided a restraining device comprising a support incorporating a handle and spaced apart first and second anchor points, a strap being attached to the first anchor point and slidably attached to the second anchor point, the strap and the support defining a restraining enclosure to enclose a detainee, wherein in use, movement of the strap in a forward direction reduces the area of the enclosure and movement of the strap in a reverse direction increases the area of the enclosure, characterised in that said support defines, between said anchor points, an elongate abutment surface forming a front face for engagement with a torso or limb of a detainee.

Preferably, the support is substantially T-shaped, the anchor points being positioned on first and second arms of the support.

At least one arm of the support may have a hinging means. The support when stored may be box-shaped and, in use, at least one arm may be rotated around its hinging means by a usable device.

Also preferably, a cavity is defined within the second anchor point and handle of the support, the strap being slidably located within the cavity.

Again preferably, the strap is made of webbing and may be releasably attached to the first anchor point of the support, preferably, by means of a releasable pin.

The support may have a locking mechanism moveable between an open position allowing movement of the strap in the forward or reverse direction and a closed position preventing movement or the strap in the reverse direction. The locking mechanism may also be positioned in a locking space within the cavity and is preferably positioned in the handle.

The strap may have a strap handle fixed to its free end to aid movement of the strap in the forward direction. The strap may also have a clip at its free end, the clip being slidable along the strap and having a fixing means to fix the clip to the strap.

An extension handle may be attached to the handle of the support.

The support may have a front face abutting the detainee, which is curved or shaped. The front face of the support may be padded.

The restraining device of the present invention can be easily applied to the detainee, for example, whilst the detainee is standing up and maybe struggling, or from the rear before any attack commences, or even when the detainee is lying down. The restraining device can also be placed on the detainee prior to the potentially dangerous manoeuvre of placing handcuffs on the detainee, making it safer for the person applying the handcuffs. It is also safer for the detainee being handcuffed since injuries often occur to the detainee if struggling in such situations.

The restraining device according to a further embodiment of the present invention can be applied to the detainee's legs if he continues to struggle and can be tightened to encourage the detainee to lay on the ground. If the detainee continues to resist, the restraining device around the legs can be used to force the detainee to the ground by supporting the detainee and pulling his legs away. If it is necessary to move or escort the detainee, the restraining device can be loosened and be used to ensure that the detainee being moved can walk and be safely guided to a place of safety. This ensures that the person escorting the detainee, the user, can keep at a safe distance away from the legs and feet but still have control, by simply re-tightening the restraining device.

Use of the restraining device of the present invention around the upper torso and arms, and the legs in conjunction with, for example, handcuffs, makes it almost impossible for the detainee to move and, therefore, to harm or injure himself or others around.

The restraining device of the present invention, when fitted around the upper torso will restrict the movement of the detainee's arms, by holding the arms against the body. If used when the detainee is sitting on a fixed seat the restraining device can be used to secure the detainee to the seat, for example, during transportation of a prisoner or in an aircraft. The restraining device can also be used to secure the detainee to other objects, such as, a hospital bed.

In order to aid in understanding the invention some specific embodiments thereof will now be described by way of example and with reference to the accompanying drawings, in which:
Figure 1 is an angled plan view showing a restraining device of the present invention;
Figure 2 is an angled plan partly sectional view of the device of Figure 1 of the present invention with the cover plate and strap removed;
Figure 3 is an angled plan view of the components of the locking mechanism of the device of Figure 1;
Figure 4 is an angled plan view of a restraining device of a further embodiment of the present invention.

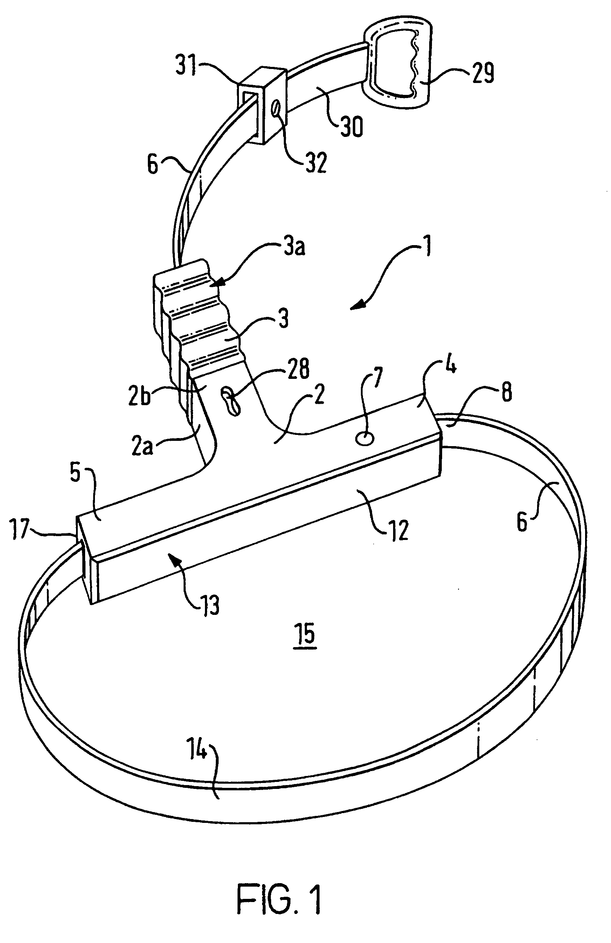

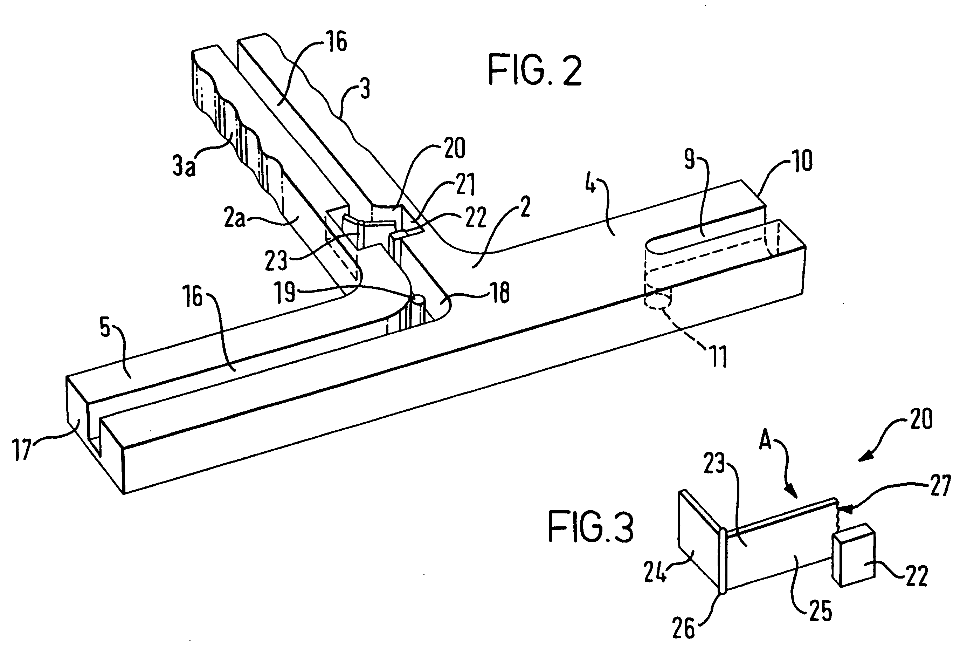

Referring to Figures 1 and 2, there is shown a restraining device 1, comprising a support 2 being substantially T-shaped and preferably comprising a base 2a and a cover plate 2b, the support 2 having a handle 3 and first and second arms 4 and 5. The handle 3 having a grip 3a, preferably, of rubber. The first and second arms 4 and 5 may be attached to the support 2 by hinging means (not shown), of conventional types. The arms 4 and 5 could then be stored by rotating the arms 4 and 5 around the hinging means (not shown) so that the length of the arms 4 and 5 come alongside the handle (3), so that the support (2) is box-shaped for storage. The hinging means may be any conventional hinge device and is preferably fitted so that the arms 4 and 5 are separated substantially near the centre of their length and a hinging means (not shown) is fitted to attach each arm 4 and 5 to the opposite end of the handle 3 from the grip 3a. Alternatively, the arms 4 and 5 may form a unitary section having one hinging means (not shown) for attachment of the arms 4 and 5 to the handle 3 of the support 2. A strap 6 being releasably fixed to the first arm 4 of the support 2 by means of a releasable pin 7. The releasable pin 7 being designed to be quickly releasable but not releasable by a detainee, for example, the pin 7 may require use of a key for its release. The strap 6 of, for example, webbing, having a fixed end 8 which is preferably contained in a slot 9 in the base 2a of the support 2 at a first anchor point or first end 10 of the first arm 4, the slot 9 having a pin hole 11 and the strap 6 being attached by means of the pin 7 entering through the pin hole 11 and through a loop (not shown) at the fixed end 8 of the strap 6 to secure the strap 6. The support 2 having a front face 12 which may be shaped or curved to which is attached a pad 13. The strap 6 having a restraining portion 14, and the restraining portion 14 and the front face 12 of the support 2 form an enclosure 15 in which an object or person (not shown) can be restrained.

The strap 6 is contained within a cavity 16 within the base 2a of the support 2. The cavity 16 running from a second anchor point or second end 17 of the second arm 5, longitudinally along substantially the centre of the second arm 17 then at a substantially right angle 18 and longitudinally along substantially the centre of the handle 3. A cog 19 is positioned in the cavity 16 at the substantially right angle 18, the cog 19 turning with the strap 6 when the strap 6 is pulled through the cavity 16 to aid movement of the strap 6 through the cavity 16. A locking mechanism 20 is contained within a locking space 21. The locking mechanism 20 comprising a fixed abutment 22 and a movable abutment 23. The movable abutment 23 comprising a first wing 24 and a second wing 25 angled around a pivot 26, the second wing 24 having a serrated edge 27 to grip the strap 6. The movable abutment 23 is biased in the direction A so that the second wing 25 abuts with the fixed abutment 22. It is obvious that other conventional locking mechanisms 20 could be used in the restraining device 1 of the present invention.

The strap 6 passes between the fixed abutment 22 and the moveable abutment 23 of the locking mechanism 20. The cover plate 2b of the support 2 has a key hole 28. The key hole 28 enables a key (not shown) to be used to lock or unlock the locking mechanism 20. The locking mechanism 20 is locked by preventing movement of the moveable abutment 23 so that the second wing 25 is fixed in a position when it abuts the fixed abutment 22. The strap 6 has a strap handle 29 at its free end 30 to aid pulling of the strap 6 to decrease the area of the enclosure 15 and, therefore, tighten the restraining device 1. A clip 31 is located on the free end 30 of the strap 6. The clip 31 is slidable along the strap 6 and has a fixing means or screw 32 to fix the clip to the strap at any position. The clip 31 can be used to further secure the strap 6 when a person is being detained. In a non illustrated variant, rather than being free to slide along the strap 6, the clip 31.may be integral with the handle 3.

The arms 4 and 5 may be rotated around the hinging means (not shown) by a usable device (not shown). The usable device (not shown) may comprise a conventional spring or lever mechanism. The handle 3 may have mechanical releasing mechanism (not shown) for operation of the usable device (not shown) and it is foreseen that the handle 3 and mechanical releasing mechanism could form a pistol grip. Preferably, activation of the usable device by means of the mechanical releasing mechanism for rotation of the arms 4 and 5 into operational position would also extend the strap 6.

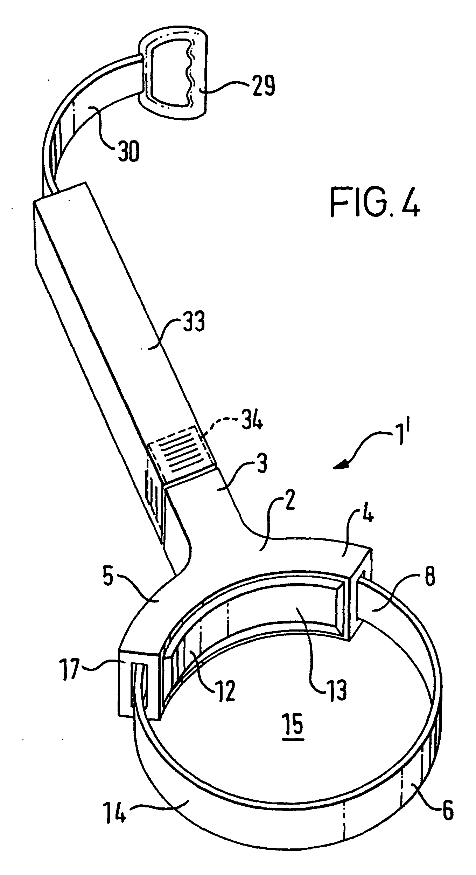

Referring to Figure 4, there is shown a further embodiment of the restraining device 1' of the present invention, specifically designed for use to restrain a detainee around the legs. The front face 2 is curved to enable the device 1' to be positioned around the detainee's legs (not shown) more easily. An extension handle 33 is attached by any conventional attachment means 34 to the handle 3 of the support 2. The strap 6 running through the cavity 16 in the extension handle 33. The extension handle 33 enables the detainee's legs to be restrained while the user of the device 1' can keep a safe distance from the detainee's legs. Obviously, the extension handle 33 can be attached to any modification of the restraining device 1' and is particularly of use when escorting a detainee, again enabling the user of the device 1' to keep a safe distance from the detainee.

In use, the restraining portion 14 of the strap 6 is placed over the detainee's head so that the detainee's upper torso and arms are within the enclosure 15 and the restraining portion 14 of the strap 6 is around the lower part of the detainee's upper arm. The front face 12 of the support 2 is positioned behind the detainee. The user of the restraining device 1, holds the support 2 by means of the grip 3a of the handle 3 and pulls the strap 6 by means of the strap handle 29. Pulling of the strap 6 by means of the strap handle 29 pulls the strap 6 through the cavity 16 and, therefore, reduces the length of strap 6 in the restraining portion 14 and the area of the enclosure 15, causing the restraining device to tighten around the detainee. The detainee's arms are then held against his body.

The strap 6 passes freely through the locking mechanism 20 as the strap 6 is pulled in the forward or tightening direction. However, should the detainee struggle and attempt to loosen the strap 6, the serrated edge 27 will grip the strap 6 causing the second wing 25 of the moveable abutment 23 to abut tightly against the fixed abutment 22 therefore preventing movement of the strap in the reverse or loosening direction. The locking mechanism 20 can be locked in an open or closed position allowing free movement of the strap 6 in either direction or preventing movement of the strap 6 in the reverse direction, respectively. The locking mechanism 20 is opened or closed by means of a key (not shown) inserted in the key hole 28 in the cover plate 2b. The strap 6 can be further secured by means of the clip 31 at the free end 30 of the strap 6, being positioned near the handle 3 of the support 2 for extra security.

For quick release in an emergency or if the detainee is still violent and has to be placed into a cell or room. The releasable pin 7 can be released and removed from the pin hole 11 which releases the fixed end 8 of the strap 6. Normal release is effected by opening the locking mechanism 20 so that the strap 6 is free to move in either direction.

The restraining device 1' as shown in Figure 4 is used in the same way as mentioned above, however, the locking mechanism 20 may be released by a trigger rather than the key, to enable the user to keep a distance from the detainee's legs and feet. The restraining device 1' as shown in Figure 4 is used to secure the legs together above the ankles by placing the strap 6 over the feet up to the ankles and pulling the strap 6 by means of the strap handle 29 to reduce the area of the enclosure 15. This device 1' can also be used to control the detainee during escort. The strap 6 is loosened, but left around the detainee's legs, allowing the detainee to walk at the user's direction. It is advantageous to use a restraining device 1' around the detainee's upper torso and arms, at the same time as using a device 1' around their legs, therefore, if the detainee being escorted becomes violent and refuses to comply, the device around the legs can be tightened and the detainee can be assisted to the ground by the user holding the device 1' around the upper torso and arms of the detainee. There is complete control when escorting a violent detainee and it requires no physical contact by the users escorting, saving harm or injury to users and also helping prevent any risk of catching a disease or infection from the detainee being escorted.

The restraining device of the present invention is applicable to many situations, for example, aircraft/public transport, law enforcement, medical treatment, rescue and public places.

If a passenger becomes violent or is threatening or acting in a violent manner in an aircraft, where the cabin crew believe that the passenger is endangering the safety of the aircraft and the passengers. The fact that an offence is being or about to be committed can be reported to the Captain of the aircraft. Then, if normal verbal persuasion fails or if the passenger is already violent, the restraining device of the present invention could be applied around the upper torso and arms of the passenger to restrain him, or around the passenger and the seat to secure the passenger to the seat. Once the passenger has been secured a further restraining device should be applied to the legs, and this further restraining device could be tightened securely if necessary. In such a situation, if the passenger remains violent then normal handcuffs can be applied more easily and without risk of harm or injury to the person applying them.

Once secured if the captain decides that the passenger is still causing harm or injury to the other passengers and staff, the passenger can be moved to a holding area (for example, toilets or crew seats) in a safe and easy manner, by using the restraining devices around the upper torso and around the legs, without the risk of danger or injury to staff or other passengers. If necessary, the passenger can be restrained on the aircraft floor by fewer staff, than if he was only handcuffed or unrestrained.

It is obvious that the same principle and application of the restraining means of the present invention can be used on all other forms of public transport with modifications varied for size and design of seating.

The restraining device of the present invention could also be used by Police Officers attempting to arrest or detain a person who is violent, without risk of harm or injury to themselves or the detained person.

After a person has been arrested there is the question of transportation. There are two main concerns when transporting, violent or potentially violent prisoners. First is the protection of the Police Officers and their equipment, such as their vehicles, custody rooms etc.. The other is to restrain the prisoner so he cannot cause undue harm or injury to himself, unruly or violent prisoners are extremely difficult to handle which often results in injury to the police officer or the prisoner.

If the restraining device of the present invention is applied together with any form of handcuffs, it is almost impossible for the prisoner to injure himself, provided the head is protected by being held or secured on the ground avoiding the prisoner's mouth.

The restraining device of the present invention could also be used on violent psychiatric or delirious patients refusing treatment and being violent against a member of medical staff, for example, to secure injured drug addicts or drunks to stretchers to avoid injury to the detainee and the paramedics/ambulance crews.

The restraining device of the present invention could also be used to secure a person being rescued, who is panicking possibly without knowing, placing their rescuer in physical danger, for example, a person drowning will often panic and try to hold on to the rescuer causing further danger to both the rescuer and the person being rescued.

It will be understood that various alterations and modifications may be made to the above embodiments without departing from the scope of the invention, and that the invention is applicable to many different situations, in particular, the device could be used for restraining animals as well as humans. The device is preferably made in the form of a base 2a and a cover plate 2b, however, obviously the device could be made by moulding a single support 2 or by moulding two equal halves. The front face 12 of the support 2 can be flat or curved according to the desired use of the device 1. Also, it is understood that any other conventional locking mechanism 20 could be used. Further, the restraining device of the present invention could be battery or motor driven and involve a clutching mechanism and/or a torsion control device.

## Claims

1. A restraining device comprising a support (2) incorporating a handle (3) and spaced apart first and second anchor points (4,5,10,17), a strap (6) being attached to the first anchor point (4,10) and slidably attached to the second anchor point (5,17), the strap (6) and the support (2) defining a restraining enclosure (15) to enclose a detainee, wherein in use, movement of the strap (6) in a forward direction reduces the area of the enclosure (15) and movement of the strap (6) in a reverse direction increases the area of the enclosure (15), **characterised in that** said support (2) defines, between said anchor points an elongate abutment surface forming a front face (12) for engagement with a torso or limb of a detainee.

2. A restraining device as claimed in Claim 1, wherein the support (2) is substantially T-shaped and the anchor points (10,17) are positioned on first and second arms (4,5) of the support (2).

3. A restraining device as claimed in Claim 2, wherein at least one arm (4,5) is hinged.

4. A restraining device as claimed in Claim 3, wherein the support (2) is box-shaped when stored and, in use, at least one arm (4,5) is rotated around its hinge by a spring means.

5. A restraining device as claimed in any preceding claim, wherein a cavity (16) is defined within the second anchor point (5,17) and the handle (3) of the support (2), the strap (6) being slidably located within the cavity (16).

6. A restraining device as claimed in any preceding claim, wherein the strap (6) is made of webbing.

7. A restraining device as claimed in any preceding claim, wherein the strap (6) is releasably attached to the first anchor point (4,10) of the support (2), by means of a releasable pin (7).

8. A restraining device as claimed in any one of Claims 5 to 7, wherein a locking mechanism (20) is located within a locking space (21) within the cavity (16), the locking mechanism (20) being moveable between an open position allowing movement of the strap (6) in the forward or reverse direction and a closed position preventing movement of the strap (6) in the reverse direction.

9. A restraining device as claimed in any preceding claim, wherein the front face (12) which, in use, abuts the detainee, is curved or shaped, and/or is padded (13).

## Patentansprüche

1. Eine Fesselvorrichtung bestehend aus einer Stütze (2) mit Griff (3) und in bestimmten Abständen voneinander angeordneten ersten und zweiten Ankerpunkten (4,5,10,17), wobei besagte Stütze (2) zwischen besagten Ankerpunkten einen längeren Bereich aufweist, der eine Stirnfläche (12) für den Kontakt zum Körper bzw. Körperglied eines Häftlings darstellt. Ein Riemen (6) wird dabei am ersten Ankerpunkt (4,10) und am zweiten Ankerpunkt (5,17) befestigt, wobei sich seine Position hier verschieben lässt. Riemen (6) und Stütze (2) bilden eine Körperfessel (15) für den Häftling, wobei bei der Verwendung eine Bewegung des Riemens (6) nach vorne die Fesseln (15) weiter festziehen und eine Bewegung des Riemens (6) in die andere Richtung die Fesseln (15) lockern wird.

2. Eine Fesselvorrichtung gemäß Anspruch 1, wobei die Stütze (2) im Wesentlichen T-förmig ist und die Ankerpunkte (10,17) am ersten und zweiten Arm (4,5) der Stütze (2) positioniert sind.

3. Eine Fesselvorrichtung gemäß Anspruch 2, wobei mindestens ein Arm (4,5) ein Scharnier umfasst.

4. Eine Fesselvorrichtung gemäß Anspruch 3, wobei die Stütze (2) bei der Aufbewahrung kastenförmig ist und bei einer Verwendung mindestens ein Arm (4,5) durch eine Feder um das Scharnier rotiert wird.

5. Eine Fesselvorrichtung gemäß jeglicher vorangehender Ansprüche, wobei sich ein Hohlraum (16) im zweiten Ankerpunkt (5,17) und dem Griff (3) der Stütze (2) befindet und der Riemen (6) im Hohlraum (16) platziert wird, wobei sich seine Position verschieben lässt.

6. Eine Fesselvorrichtung gemäß jeglicher vorangehender Ansprüche, wobei es sich beim Riemen (6) um ein Gurtband handelt.

7. Eine Fesselvorrichtung gemäß jeglicher vorangehender Ansprüche, wobei der Riemen (6) mittels eines lösbaren Stifts (7) am ersten Ankerpunkt (4,10) der Stütze (2) befestigt wird und gelöst werden kann.

8. Eine Fesselvorrichtung gemäß einem der Ansprüche 5 bis 7, wobei eine Verriegelung (20) innerhalb eines speziell dafür vorgesehenen Bereichs (21) im Hohlraum (16) positioniert ist. Die Verriegelung (20) ermöglicht dabei im geöffneten Zustand eine Bewegung des Riemens (6) in Vor- oder Rückwärtsrichtung und verhindert im geschlossenen Zustand eine Bewegung des Riemens (6) in Rückwärtsrichtung.

9. Eine Fesselvorrichtung gemäß jeglicher vorangehender Ansprüche, wobei die Stirnfläche (12), die den Kontakt zum Häftling darstellt, bogenförmig ist oder eine bestimmte körperangepasste Form hat sowie/oder gepolstert (13) ist.

## Revendications

1. Un dispositif d'immobilisation comprenant un support (2) se composant d'une poignée (3) et, espacés l'un de l'autre, un premier et un deuxième point d'ancrage (4,5,10,17), ledit support (2) formant, entre lesdits points d'ancrage, une surface de butée allongée formant une face antérieure (12) qui se place contre le torse ou un des membres du détenu, une courroie (6) étant fixée sur le premier point d'ancrage (4,10) et, par coulissement, sur le deuxième point d'ancrage (5,17), la courroie (6) et le support (2) formant une enceinte d'immobilisation (15) entourant le détenu, de sorte qu'en cours d'usage, le mouvement en avant de la courroie (6) réduit la superficie de l'enceinte (15), alors que le mouvement de la courroie (6) en arrière augmente la superficie de l'enceinte (15) .

2. Un dispositif d'immobilisation conforme à la revendication 1 dans lequel le support (2) est quasiment en forme de T, et les points d'ancrage (10,17) sont positionnés sur le premier et le deuxième bras (4,5) du support (2).

3. Un dispositif d'immobilisation conforme à la revendication 2, dans lequel au moins un bras (4,5) est articulé.

4. Un dispositif d'immobilisation conforme à la revendication 3 dans lequel le support (2) est en forme de boîte lorsqu'il est rangé et, en cours d'usage, au moins un des bras (4,5) pivote sur son axe sous l'action d'un ressort.

5. Un dispositif d'immobilisation conforme à la revendication précédente dans lequel une cavité (16) se forme dans le deuxième point d'ancrage (5,17) et la poignée (3) du support (2), la courroie (6) coulissant au sein de la cavité (16) .

6. Un dispositif d'immobilisation conforme à la revendication précédente dans lequel la courroie (6) est fabriquée en toile forte.

7. Un dispositif d'immobilisation conforme à la revendication précédente dans lequel la courroie (6) se fixe, de façon amovible, sur le premier point d'ancrage (4,10) du support (2), au moyen d'une cheville détachable (7).

8. Un dispositif d'immobilisation conforme à une quelconque des revendications 5 à 7, dans lequel un mécanisme de verrouillage (20) se place dans un évidement de verrouillage (21) dans la cavité (16), ce mécanisme de verrouillage (20) pouvant être déplacé entre une position ouverte, permettant le déplacement de la courroie (6) en avant ou en arrière, et une position fermée, empêchant le déplacement en sens inverse de la courroie (6).

9. Un dispositif d'immobilisation conforme à une quelconque des revendications précédentes, dans lequel la face antérieure (12) qui, en cours d'usage, vient buter contre le détenu, est courbe ou façonnée et/ou rembourrée (13).
